(19) Europäisches Patentamt
European Patent Office
Office européen des brevets

(11) Publication number: 0 068 822 A2

(12) EUROPEAN PATENT APPLICATION

(21) Application number: 82303296.6

(22) Date of filing: 24.06.82

(51) Int. Cl.³: C 07 D 207/44, C 07 D 207/452, C 07 D 209/46, C 07 D 209/48, C 07 D 471/04, C 07 D 487/04, C 07 D 491/052, C 07 D 495/04, A 01 N 43/36, A 01 N 43/38, A 01 N 43/52
// (C07D471/04, 235/00, 221/00), (C07D487/04, 249/00, 237/00), (C07D491/052, 311/00, 209/00), (C07D495/04, 333/00, 209/00)

(30) Priority: 29.06.81 US 278241
06.05.82 US 375248

(43) Date of publication of application: 05.01.83
Bulletin 83/1

(84) Designated Contracting States: AT BE CH DE FR GB IT LI NL SE

(71) Applicant: Rohm and Haas Company, Independence Mall West, Philadelphia, Pennsylvania 19105 (US)

(72) Inventor: Swithenbank, Colin, 1710 West Rock Road, Perkasie Pennsylvania 18944 (US)

(74) Representative: Wood, Peter Worsley Spencer et al, Rohm and Haas Company Patent Department Chesterfield House Barter Street, London, WC1A 2TP (GB)

(54) **Novel heterocyclic substituted benzenes, herbicidal compositions containing them and the use thereof for combating weeds.**

(57) Compounds of the formula below are disclosed as selective herbicides:

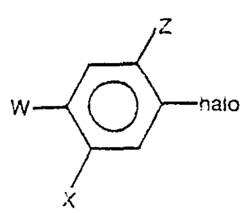

where W is a substituted heterocyclic selected from:

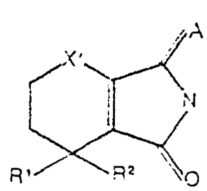

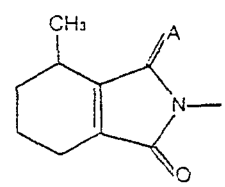

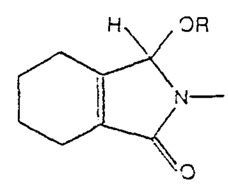

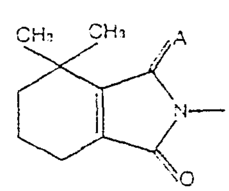

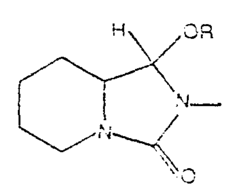

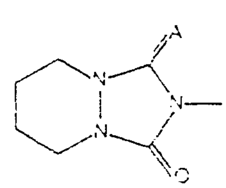

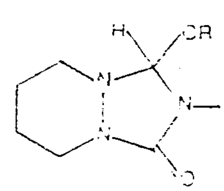

EP 0 068 822 A2

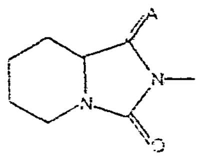 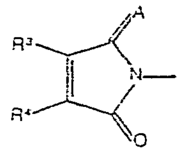

wherein A is O or S; halo is chloro, bromo or fluoro; $R^1$ and $R^2$ are the same or different radicals selected from hydrogen or $(C_1-C_4)$ alkyl; X is hydrogen or halo; $X^1$ is methylene, O or S; Z is carboxy, alkoxyalkoxy, alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy, alkoxycarbonylalkenyloxy, a 5- to 6-membered heterocyclic, formylalkoxy, haloalkoxy, mononuclear arylcarbonylalkoxy, oxiranylalkoxy, amino, monoalkyl amino, dialkylamino, hydrazino, monoalkylhydrazino, dialkylhydrazino, acylamino, alkoxyamino, alkoxycarbonylamino, mono- or dialkylcarbonylamino, N-alkyl-N-alkoxyalkylaminocarbonylamino, haloalkylcarbonylamino, N-alkylsulfonylaminocarbonyl, N-alkylsulfonylamino, monoalkenylamino, dialkenylamino, monoalkynylamino, dialkynylamino, N-alkyl-N-alkenylamino, or N-alkyl-N-alkynylamino; R is hydrogen or $(C_1-C_4)$alkyl and $R^3$ and $R^4$ are the same or different and are selected from hydrogen, $(C_1-C_4)$-alkyl, $(C_3-C_5)$alkenyl or $(C_3-C_5)$alkynyl.

NOVEL HETEROCYCLIC SUBSTITUTED BENZENES, HERBICIDAL COMPOSITIONS CONTAINING THEM AND THE USE THEREOF FOR COMBATING WEEDS

This invention concerns the provision of new heterocyclic substituted benzenes, herbicidal compositions containing them and methods of combating weeds. The search has continued for even better and more selective herbicides. An ideal herbicide should give selective weed control, over a full growing season, with a single administration at a low rate of application of the herbicide. The ideal herbicide should be able to control all common weeds by killing them either as the seed, the germinating seed, the seedling or the growing plant. At the same time the ideal herbicide should be substantially non-phytotoxic to the crop or crops to which it is applied. Furthermore, the ideal herbicide should be decomposable or otherwise be dissipiated so as not to poison the soil for any long period of time. The ideal herbicide has yet to be discovered. The methods employed for killing weeds usually involves rotating the herbicides or by employing mixtures of herbicides in order to broaden the spectrum of weeds that may be controlled. Applicants have discovered novel compounds which can be added to the existing arsenal.

The following patents disclose miscellaneous herbicidal heterocyclic compounds: Netherlands Patent Specification Nos. 7117690, 7507233 and 8002020; Japanese Patent Specification Nos. 53073557, 50142730, 52093544, 48099338 and 9006121; Belgian Patent 862844, German Patent Specification Nos. 2604989, 2831770 and U.S. Patent 3987057. Netherlands Patent Specification

No. 8002020 discloses some of the starting materials employed to prepare some of the novel compounds of this invention.

In accordance with the present invention, there are provided phthalimides having the following structural formula:

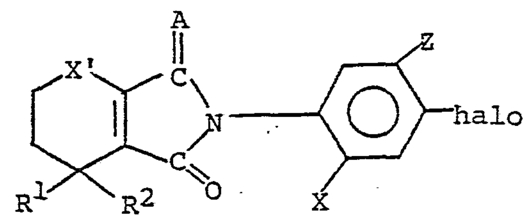

(I)

wherein A is O or S; $X^1$ is $-CH_2-$, O or S; $R^1$ and $R^2$ are the same or different and are selected from hydrogen or $(C_1-C_4)$ alkyl; X is hydrogen or halo; particularly bromo or fluoro; halo is chloro, bromo or fluoro and Z is carboxy, alkoxycarbonyl (for example, alkoxycarbonyl having up to 4 carbon atoms in the alkoxy moiety such as methoxycarbonyl, ethoxycarbonyl, propoxycarbonyl and butoxycarbonyl), alkoxycarbonylalkoxycarbonyl (for example alkoxycarbonylalkoxycarbonyl - having in each alkoxy moiety - which may be the same or different - 1, 2, 3 or 4 carbon atoms such as methoxycarbonylmethoxycarbonyl, ethoxycarbonylpropoxycarbonyl, methoxycarbonyl-1-methylmethoxycarbonyl, ethoxycarbonyl-1-methylmethoxycarbonyl or ethoxycarbonyl-1-ethylmethoxycarbonyl); acyloxy, for example, alkanoyloxy having up to 4 carbon atoms in the alkyl moiety such as acetyloxy, propionyloxy, butanoyloxy; acylalkoxy, for example, alkanoylalkoxy having up to 4 carbon atoms in each of the alkanoyl and alkoxy groups such as acetylmethoxy or propionylethoxy; cyanoalkoxy for example cyano$(C_1-C_4)$alkoxy such as cyanomethoxy,

cyanoethoxy, cyanopropoxy or cyanobutoxy or cyano-1-methylmethoxy; alkylthioalkoxy, for example, $(C_1-C_4)$alkylthio$(C_1-C_4)$alkoxy such as methylthio-methoxy, ethylthioethoxy or propylthiopropoxy; alkoxycarbonylalkoxy, for example, alkoxycarbonylalkoxy having 1 to 4 carbon atoms in each alkoxy moiety such as methoxycarbonylmethoxy, ethoxycarbonyl-ethoxy or propoxycarbonylmethoxy; alkoxycarbonylalkenyloxy, for example, alkoxycarbonylalkenyloxy having up to 4 carbons in each of the alkoxy and alkenyloxy moieties such as methoxycarbonyl-2-propenyloxy; a 5- to 6-membered heterocyclic ring having from 1 to 3 heteroatoms selected from O, N or S such as 4-morpholino, 1-piperidyl, 3-thiazinyl, 1-(4-methylpiperazinyl); formylalkoxy, for example, formyl$(C_1-C_4)$alkoxy such as formylmethoxy, formylethoxy, formylpropoxy or formylbutoxy; haloalkoxy, for example, halo$(C_1-C_4)$alkoxy such as chloroethoxy, bromoethoxy, fluoroethoxy or chlorobutoxy; mono-nuclear arylcarbonylalkoxy preferably having up to 4 carbon atoms in the alkoxy moiety, for example, benzoyl$(C_1-C_4)$alkoxy such as benzoylmethozy or benzoylethoxy; oxiranylalkoxy, for example, oxiranyl$(C_1-C_4)$alkoxy such as oxiranylmethoxy or oxiranylethoxy; amino, mono- or dialkylamino, such as mono- or di- $(C_1-C_4)$alkylamino, hydrazino, mono- or di-alkylhydrazino such as mono- or di-$(C_1-C_4)$alkyl-hydrazino; acylamino such as alkanoylamino, for example, alkanoylamino having up to 4 carbon atoms in the alkyl moiety; alkoxyamino, such as $(C_1-C_4)$alkoxyamino; alkoxycarbonylamino, such as alkoxycarbonylamino having up to 4 carbon atoms in the alkoxy moiety; mono- or dialkylaminocarbonylamino, such as mono- or di-$(C_1-C_4)$-alkylaminocarbonylamino; N-alkyl-N-$(C_1-C_4)$alkoxy-$(C_1-C_4)$-alkylaminocarbonylamino; haloalkylcarbonylamino

particularly having up to 4 carbon atoms in the alkyl moiety; such as chloroalkyl($C_1$-$C_4$)carbonylamino; N-alkylsulfonylaminocarbonyl, such as N-($C_1$-$C_4$)alkylsulfonylaminocarbonyl; alkoxyalkoxycarbonyl, such as alkoxy($C_1$-$C_4$)alkoxy($C_1$-$C_4$)carbonyl; alkoxyalkoxy such as alkoxy($C_1$-$C_4$)alkoxy($C_1$-$C_4$); N-alkylsulfonylamino, such as N-($C_1$-$C_4$)alkylsulfonylamino;mono- or dialkenylamino, such as mono- or di- ($C_3$-$C_5$)alkenylamino; mono- or di-alkynylamino, such as mono- or di-($C_3$-$C_5$)alkynylamino; N-alkyl-N-alkenylamino, such as N-($C_1$-$C_4$)alkyl-N-($C_3$-$C_5$)alkenylamino and N-alkyl(eg $C_1$-$C_4$)-N-alkynyl(eg $C_3$-$C_5$)amino. Also included, when Z is carboxy, are the agronomically acceptable salts, esters (ie other esters in addition to those already specified) and amides which are considered to be functionally equivalent to the parent compounds. For example, when Z is carboxy, salts such as the alkali metal and alkaline earth metal salts and amides, for example, ($C_1$-$C_4$)alkyl and di($C_1$-$C_4$)alkylamides are considered to be functionally equivalent to the carboxy radical.

Exemplary compounds of Formula I are those in which X' is O or S and $R^1$ and $R^2$ are both hydrogen.

The aliphatic substituents in the compounds of Formula I may be straight or branched chain. The term halo used herein means chloro, bromo, fluoro and iodo unless otherwise defined. Preferably halo is bromo or chloro.

The preferred compounds of this invention are those of the formula:

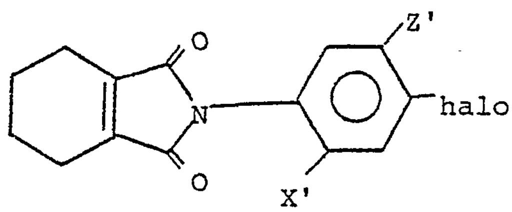

Ia

wherein halo is bromo or chloro, X' and Z' are the same or different and are selected from alkoxy($C_1$-$C_4$)-carbonyl, alkoxy($C_1$-$C_4$)alkoxy($C_1$-$C_4$)carbonyl, cyano-($C_1$-$C_4$)alkoxy, alkyl($C_1$-$C_4$)thioalkoxy($C_1$-$C_4$), acetyl-($C_1$-$C_4$)alkoxy, alkoxy($C_1$-$C_4$)alkoxy($C_1$-$C_4$), halo($C_1$-$C_4$)-alkoxy, formyl($C_1$-$C_4$)alkoxy, mono($C_1$-$C_4$)alkylamino. These compounds exhibit not only herbicidal activity, but, more importantly, exhibit desired selectivity.

Most preferred are compounds wherein Z' is cyano, ($C_1$-$C_4$)alkoxy, alkyl($C_1$-$C_4$)thioalkoxy($C_1$-$C_4$) or acetyl($C_1$-$C_4$)alkoxy, and X is hydrogen.

Examples of preferred compounds are as follows:

N-[3-(methoxycarbonyl)-4-chlorophenyl]-3,4,5,6-tetrahydrophthalimide;

N-[3-(1-methoxycarbonylethoxycarbonyl)-4-chlorophenyl]-3,4,5,6-tetrahydrophthalimide;

N-[3-(2-methoxycarbonylethoxycarbonyl)-4-chlorophenyl]-3,4,5,6-tetrahydrophthalimide;

N-[3-cyanomethoxy-4-chlorophenyl]-3,4,5,6-tetrahydrophthalimide;

N-[3-(1-cyanoethoxy)-4-chlorophenyl]-3,4,5,6-tetrahydrophthalimide;

N-[3-(1-cyanopropionoxy)-4-chlorophenyl]-3,4,5,6-tetrahydrophthalimide;

N-[3-(acetylmethoxy)-4-chlorophenyl]-3,4,5,6-tetrahydrophthalimide;

N-[3-(1-acetylethoxy)-4-chlorophenyl]-3,4,5,6-tetrahydrophthalimide;

N-[3-(methylthiomethoxy)-4-chlorophenyl]-3,4,5,6-tetrahydrophthalimide;

N-[3-(1-methylthioethoxy)-4-chlorophenyl]-3,4,5,6-tetrahydrophthalimide;

N-[3-(methoxymethoxy)-4-chlorophenyl]-3,4,5,6-tetrahydrophthalimide;

N-[3-(1-methoxyethoxy)-4-chlorophenyl]-3,4,5,6-tetrahydrophthalimide;

N-[3-[2-(1-methoxy)propionoxy]-4-chlorophenyl]-3,4,5,6-tetrahydrophthalimide;

N-[3-(formylmethoxy)-4-chlorophenyl]-3,4,5,6-tetrahydrophthalimide;

N-[3-(1-formylethoxy)-4-chloropehnyl]-3,4,5,6-tetrahydrophthalimide; and

N-[3-(N'-ethylamino)-4-chlorophenyl]-3,4,5,6-tetrahydrophthalimide.

Other compounds embraced by this invention include:

N-[3-(N'-methyl-N'-ethylamino)-4-chlorophenyl]-3,4,5,6-tetrahydrophthalimide;

N-[3-(N'-allylamino)-4-chlorophenyl]-3,4,5,6-tetrahydrophthalimide;

N-[3-(N'-methyl-N'-allylamino)-4-chlorophenyl]-3,4,5,6-tetrahydrophthalimide;

N-[3-(N'-ethyl-N'-allylamino)-4-chlorophenyl]-3,4,5,6-tetrahydrophthalimide;

N-[3-(N'propynylamino)-4-chlorophenyl]-3,4,5,6-tetrahydrophthalimide;

N-[3-(N'methyl-N'-propynylamino)-4-chlorophenyl]-3,4,5,6-tetrahydrophthalimide;

N-[3-N'-(2-methoxyethylamino)-4-chlorophenyl]-3,4,5,6-tetrahydrophthalimide;

N-[3-N'-methyl-N'-(2-methoxyethylamino)-4-chlorophenyl] 3,4,5,6-tetrahydrophthalimide;

N-[3-N'-2-(1-methoxyethylamino)-4-chlorophenyl]-3,4,5,6-tetrahydrophthalimide;

N-[3-N'-(acetylmethylamino)-4-chlorophenyl]-3,4,5,6-tetrahydrophthalimide;

N-[3-N'-(1-acetylethylamino)-4-chlorophenyl]-3,4,5,6-tetrahydrophthalimide;

N-[3-N'-methyl-N'-(acetylmethylamino-4-chlorophenyl]-3,4,5,6-tetrahydrophthalimide;

N-[3-N'-(cyanomethylamino)-4-chlorophenyl]-3,4,5,6-tetrahydrophthalimide;

N-[3-N'-(1-cyanoethylamino)-4-chlorophenyl]-3,4,5,6-tetrahydrophthalimide;

N-[3-N'-methyl-N'-(cyanomethylamino)-4-chlorophenyl]-3,4,5,6-tetrahydrophthalimide;

N-[3-N'-(methylthiomethylamino)-4-chlorophenyl]-3,4,5,6 tetrahydrophthalimide;

N-[3-N'-(1-methylthioethylamino)-4-chlorophenyl]-3,4,5,6 tetrahydrophthalimide; and

N-[3-N'-methyl-N'-(methylthiomethylamino)-4-chlorophenyl]-3,4,5,6-tetrahydrophthalimide.

Other compounds falling within the scope of this invention are illustrated by the following structural formulae.

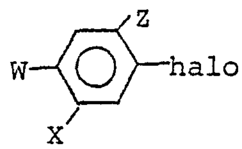 where W is substituted heterocylic of the formulae below and which are referred to herein as Formula II Compounds.

<u>Formula II Compounds</u>

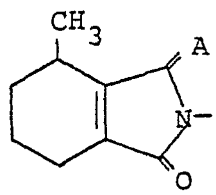

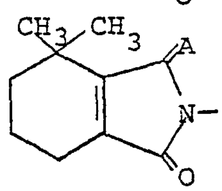

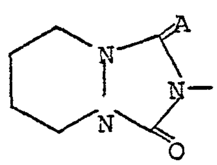

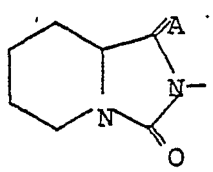

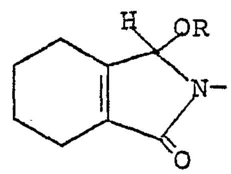

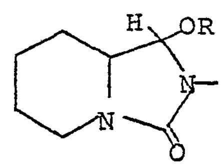

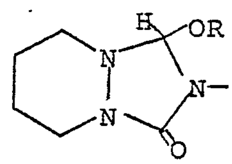

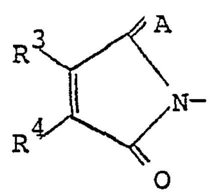

wherein A is O or S, R is hydrogen or $(C_1-C_4)$alkyl, $R^3$ and $R^4$ are the same or different and are selected from hydrogen, $(C_1-C_4)$alkyl, $(C_3-C_5)$alkenyl or $(C_3-C_5)$alkynyl.

The compounds of this invention may be prepared by one of several routes depending on the particular heterocyclic desired and also on the nature of the Z substituent. The following process illustrates the preparation of the most preferred compounds:

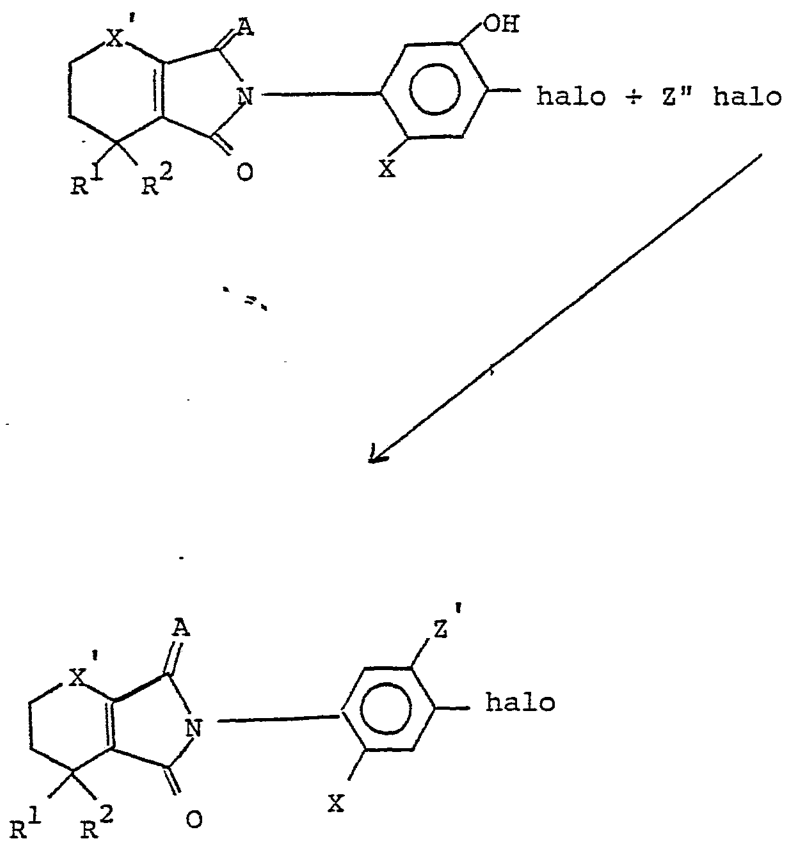

wherein A is O or S; X' is $-CH_2-$ O or S, $R^1$ and $R^2$ are the same or different and are selected from hydrogen or $(C_1-C_4)$alkyl; Z' is Z"O which is acyloxy, alkoxyalkoxy, acylalkoxy, cyanoalkoxy, alkylthioalkoxy, alkoxycarbonylalkoxy, alkoxycarbonylalkenylalkoxy, formylalkoxy, haloalkoxy, oxiranylalkoxy or arylcarboonylalkoxy. This reaction is generally conducted at a temperature in the range of from about room temperature (ca 20°C.) to about 100°C in a suitable inert polar solvent such as acetone, methylethylketone, acetonitrile, dimethyl formamide, dimethyl sulfoxide and dimethyl acetamide.

The other procedure for preparing those compounds wherein Z contains a carbonyl radical joined to the benzene ring is by treating an appropriately substituted anhydride with an appropriately substituted 3-aminobenzene compound. This reaction is generally conducted at a temperature in the range of from about room temperature to about 150°C in a suitable solvent such as glacial acetic acid, propionic acid, xylene, toluene. The following equation illustrates this process wherein A, $R^3$, $R^4$ and X are as defined above, Y is halo and Z''' is carboxy, alkoxycarbonyl or alkoxycarbonylalkoxycarbonyl.

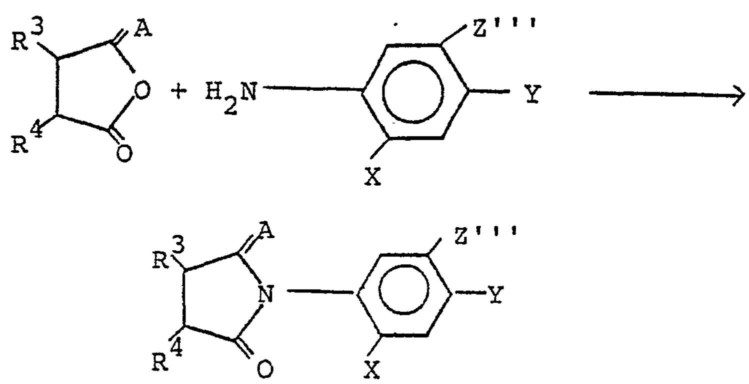

wherein $R^3$, $R^4$, X and Y are as defined above

Those compounds wherein the heterocyclic ring has two nitrogen atoms are prepared in the following manner:

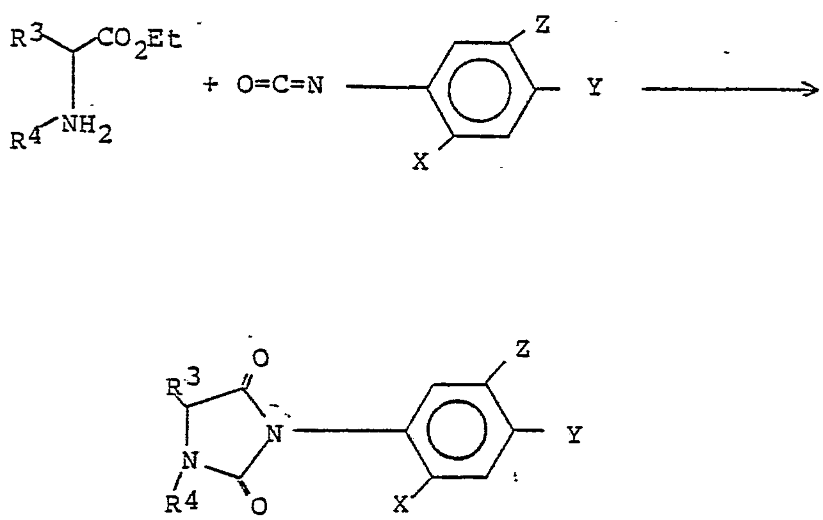

wherein $R^3$, $R^4$, X, Y and Z are as defined above. This reaction is conveniently conducted in an aqueous or alcoholic solution of a base. Examples of bases which may be employed include lithium hydroxide, sodium hydroxide and potassium hydroxide. This reaction is conducted at a temperature in the range of from about $0^o$ to about $100^oC$.

The preparation of those heterocyclic compounds containing three nitrogen atoms is illustrated by the following equation:

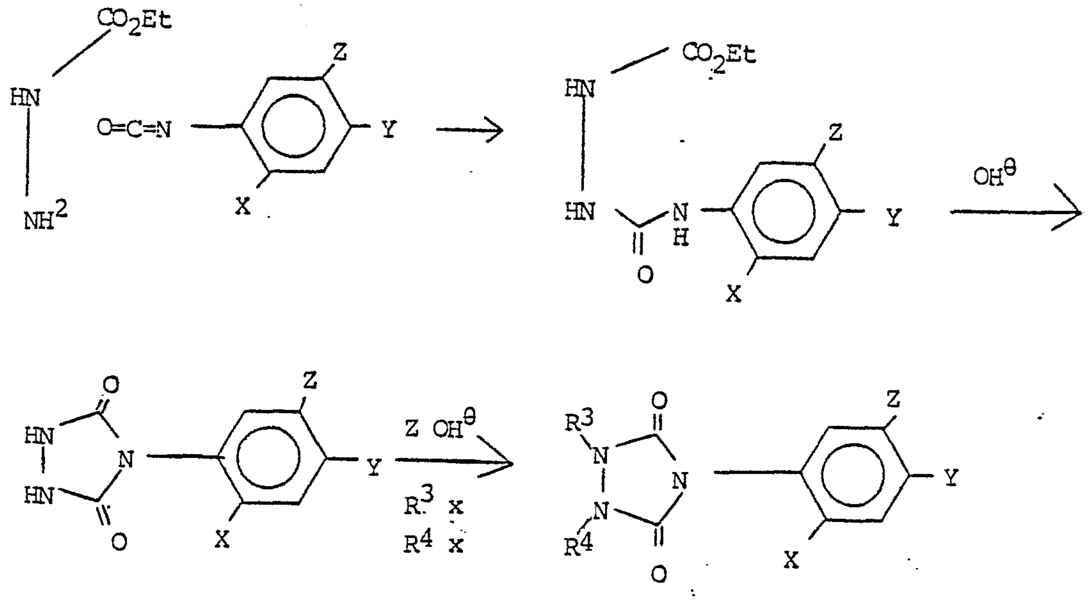


wherein $R^3$, $R^4$, X, Y and Z are as defined above. This reaction is generally conducted in the presence of an acid such as glacial acetic acid, propionic acid or p-toluene sulfonic acid in the presence of a suitable inert solvent such as ethers, aromatic solvents or halogenated hydro- carbons. The reaction is generally carried out in the temperature range of from about 0° to about 150°C.

The starting materials are either known or may be prepared by methods well known to those skilled in the art.

The following illustrates one method of preparing amino and monosubstituted amino starting materials. Subsequent reaction will afford the disubstituted amino starting materials.

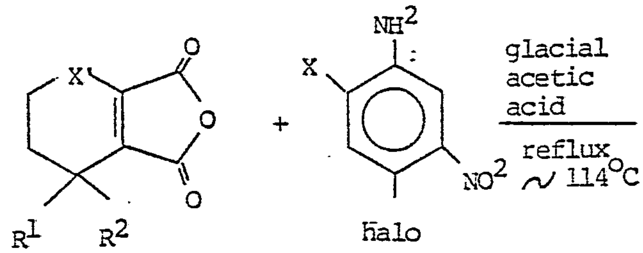
glacial
acetic
acid
reflux
~ 114°C

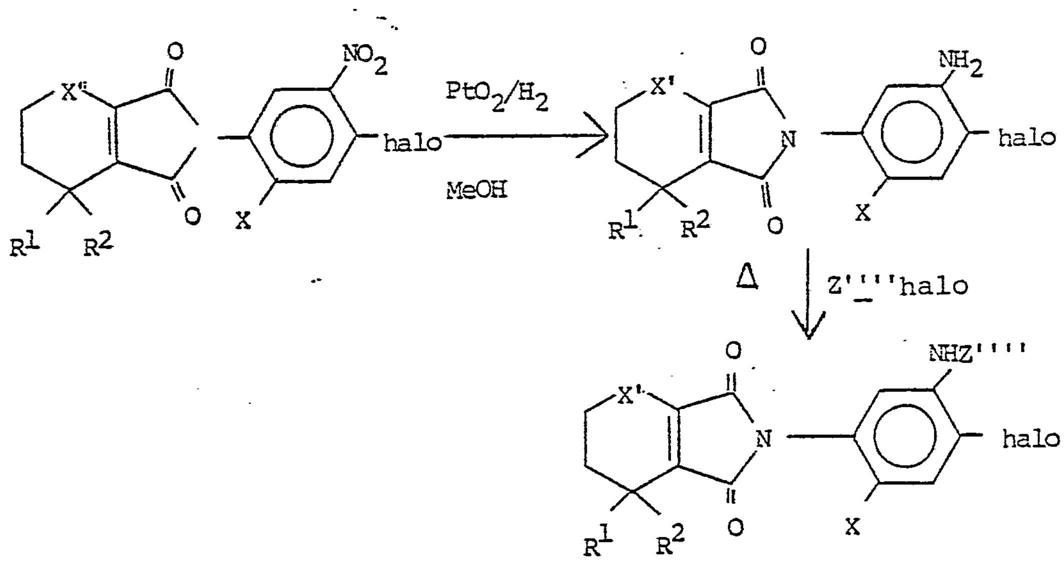
PtO2/H2
MeOH
Δ
Z''''halo

The reaction with Z'''' halo is conducted in a suitable inert solvent such as, acetonitrile, acetone, methylethylketone, dimethyl sulfoxide, dimethyl formamide or dimethyl acetamide, at a temperature in the range of from room temperature to about 150°C.

The substituted phthalimide compounds (I, supra) of the invention are useful as preemergence and postemergence herbicides. Preemergence herbicides are ordinarily used to treat the soil by application either before seeding, during seeding, or after seeding and before the crop emerges.

The compounds of this invention are especially active as pre-emergence herbicides.

Among the crops on which the compounds of the invention can be advantageously employed are: cotton, soybeans, peanuts, beans, peas, carrots, corn, wheat, rice, and other cereal crops.

The compounds (I, supra) can be applied in any amount which will give the required control of weeds. A standard rate of application of the herbicides of the invention is in the range from about 0.022 to 13.45 kg/ha (0.02 to about 12 pounds of compound per acre). A preferred range is from about 0.11 to 4.48 kg/ha (0.1 to about 4 pounds per acre).

Under some conditions, the compounds (I, supra) may be advantageously incorporated into the soil or other growth medium prior to planting a crop. This incorporation can be by any convenient means, including simple mixing with the soil, applying the compounds to the surface of the soil and then disking or dragging into the soil to the desired depth, or by employing a liquid carrier.

The substituted phthalimides (I) and the other compounds illustrated above (II) falling within the scope of the invention can be applied to the growth medium or to plants to be treated either neat or as a component of a herbicidal composition or formulation which also comprises an agronomically acceptable carrier. "Agronomically acceptable carrier" is any carrier which can be used to dissolve, disperse or diffuse a herbicidal compound in the composition without imparing the effectiveness of the herbicidal compound and which by itself has no permanent detrimental effect on the soil, equipment, crops, or agronomic environment. Mixtures of the compounds of the invention may also be used in any of these herbicidal formulations. The herbicidal compositions of the invention can be either solid or liquid formulations or solutions. For example, the compounds can be formulated as wettable powders, emulsifiable concentrates, dusts, granular formulations, aerosols, or flowable emulsion concentrates. In such formulalations, the compounds are extended with a liquid or solid carrier and, when desired, suitable surfactants are incorporated.

It is usually desirable, particularly in postemergence applications, to include adjuvants, such as wetting agents, spreading agents, dispersing agents, stickers, adhesives, and the like, in accordance with agricultural practices. Examples of adjuvants which are commonly used can be found in the John W. McCutcheon, Inc. publication "Detergents and Emulsifiers 1969 Annual."

Examples of solvents which are useful in the practice of this invention include alcohols, ketones, aromatic hydrocarbons, dimethyl formamide, dioxane, dimethyl sulfoxide, and the like. Mixtures of these

solvents can also be employed. The concentration of the solution can vary from about 2% to about 98% of active product with a preferred range being from about 25% to about 75%.

For preparation of emulsifiable concentrates, the substituted phtalimides can be dissolved in organic solvents, such as toluene, xylene, methylated naphthalene, corn oil, pine oil, isophorone, cyclohexanone, methyl oleate, and the like, or in mixtures of these solvents, together with an emulsifying agent which permits dispersion in water. Suitable emulsifiers include, ethylene oxide derivatives of alkylphenols or long-chain alcohols, mercaptans, carboxylic acids, and reactive amines and partially esterified polyhydric alcohols. Solvent-soluble sulfates or sulfonates, such as the alkaline earth salts or amine salts or alkylbenzenesulfonates and the fatty alcohol sodium sulfates, having surface-active properties can be used as emulsifiers either alone or in conjunction with an ethylene oxide reaction product. Flowable concentrates are formulated similarly to the emulsifiable concentrates and include, in addition to the above components, water and a stabilizing agent such as water-soluble salt of a poly-acrylic acid. The concentration of the active ingredient in emulsifiable concentrates is usually from about 10% to 60% and in flowable emulsion concentrates, can be as high as about 75%.

Wettable powders suitable for spraying, can be prepared by admixing the compound with a finely divided solid, such as clay, inorganic silicates and carbonates, and silicas and then incorporating wetting agents, sticking agents, and/or dispersing agents.

The concentration of active ingredients in such formulations is usually in the range of from about 20% to about 98%, and preferably, from about 40% to 75%. A dispersing agent can constitute about 0.5% to about 3% of the composition, and a wetting agent can constitute from about 0.1% to about 5% of the composition.

Dusts can be prepared by mixing the compounds of the invention with finely divided inert solids which may be organic or inorganic in nature. Materials useful for this purpose include, for example, botanical flours, silicas, silicates, carbonates and clays. One convenient method of preparing a dust is to dilute a wettable powder with a finely divided carrier. Dust concentrates containing from about 20% to about 80% of the active ingredient are commonly made and are subsequently diluted from about 1% to about 10% use concentration.

Granular formulations can be prepared by impregnating a solid, such as granular fuller's earth, vermiculite, ground corn cobs, seed hulls, including bran or other grain- hulls, or similar material. A solution of one or more of the compounds in a volatile organic solvent can be sprayed or mixed with the granular solid and the solvent then removed by evaporation. The granular material can have any suitable size eg average particle diameters in the range from 1.2 mm to 0.25 mm (= 16 to 60 mesh U.S. Bureau of Standards, Standard Screen Seives). The compounds will usually comprise from about 2 to about 15% by weight of the granular formulation.

The compounds of the invention (I and II) can also be mixed with fertilizers or fertilizing materials before their application. In one type of solid fertilizing composition in which the compounds can be used, particles of a fertilizer or fertilizing ingredients,

such as ammonium sulfate, ammonium nitrate, or ammonium phosphate, can be coated with one or more of the phthalimides The solid compounds and solid fertilizing material can also be admixed in mixing or blending equipment, or they can be incorporated with fertilizers in granular formulations. Any relative proportion of compound and fertilizer can be used which is suitable for the crops and weeds to be treated. The compound will commonly be from about 5% to about 25% of the fertilizing materials which promote the rapid growth of desired plants, and at the same time control the growth of undesired plants.

The compounds of the invention (I and II) can be applied as herbicidal sprays by methods commonly employed, such as conventional high-capacity hydraulic sprays, low-capacity sprays, airblast spray, aerial sprays, and dusts. For low volume applications, a solution of the compound is usually used. The dilution and rate of application will usually depend upon such factors as the type of equipment employed, the method of application, the area to be treated and the type and stage of development of the weeds.

For some applications, it may be desired to add one or more other herbicides along with the compounds of the invention. Examples of other herbicides which can be incorporated to provide additional advantages and effectiveness include:

Carboxylic Acids and Salts and Ester Derivatives Thereof

2,3,6-trichlorobenzoic acid, 2,3,5,6-tetrachlorobenzoic acid, 2-methoxy-3,5,6-trichlorobenzoic acid, 2-methoxy-3,5-dichlorobenzoic acid, 2-methyl-3,6-dichlorobenzoic acid, 2,3-dichloro-6-methylbenzoic acid, 2,4-dichlorophenoxyacetic acid, 2,4,5-trichlorophenoxyacetic acid, 2-methyl-4-chlorophenoxyacetic acid, 2-(2,4,5-trichlorophenoxy) propionic acid, 4-(2,4-dichlorophenoxy) butyric acid, 4-(2-methyl-4-chlorophenoxy) butyric acid, 2,3,6-trichlorophenylacetic acid, 3,6-endoxohexahydrophthalic acid, dimethyl 2,3,5,6-tetrachloroterephthalate, trichloroacetic acid, 2,2-dichloropropionic acid, 3-amino-2,5-dichlorobenzoic acid, and 2,3-dichloroisobutyric acid.

Carbamic Acid Derivatives

ethyl N,N-di(n-propylthiocarbamate, propyl N,N-di-(n-propyl) thiocarbamate, ethyl N-ethyl-N-(n-butyl) thiocarbamate, ethyl N-ethyl-N-(n-butyl) thiocarbamate, propyl N-ethyl-N-(n-butyl) thiocarbamate, 2-chloroallyl N,N-diethyldithiocarbamate, N-methyldithiocarbamic acid salts, ethyl 1-hexamethyleneiminecarbothiolate, isopropyl N-phenylcarbamate, isopropyl N(m-chloro- phenyl) carbamate, 4-chloro-2-butynyl N- (m-chloro- phenyl) carbamate, methyl N-(3,4-dichlorophenyl- carbamate, methyl-m-hydroxycarbanilate-m-methyl- carbanilate, and S-(4-chlorobenzyl)-N,N-diethylthiocarbamate.

Phenols

dinitro-o-(sec-butyl) phenol and its salts, pentachlorophenol and its salts.

## Substituted Ureas

3-(3,4-dichlorophenyl)-1,1-dimethylurea, 3-phenyl-1,1-dimethylurea, 3-(3,4-dichlorophenyl)-3-methoxy-1,1-dimethylurea, 3-(4-chlorophenyl)-3-methoxy-1,1-dimethylurea, 3-(3,4-dichlorophenyl)-1-n-butyl-1-methylurea, 3-(3,4-dichlorophenyl)-1-methoxy-1-methylurea, 3-(4-chlorophenyl)-1-methoxy-1-methylurea, 3-(3,4-dichlorophenyl)-1,1,3-trimethylurea, 3-(3,4-dichlorophenyl)-1,1-diethylurea, dichloral urea, N'[4-(2-p-methylphenyl) ethoxy] phenyl]-N-methoxy-N-methylurea,1,1,3-trimethyl-3-(5-p-chlorobenzyl-thio-1,3,4-thiadyazol-2-yl) urea, and 3-[p-chlorophenoxy) phenyl]-1,1-dimethylurea.

## Substituted Triazines

2-chloro-4,6-bis(ethylamino)-s-triazine, 2-chloro-4-ethylamino-6-isopropylamino-s-triazine, 2-methoxy-4,6-bis-(isopropylamino)-s-triazine, 2-methylmercapto-4,6-bis-(ethylamino)-s-triazine, 2-methylmercapto-4-ethylamino-6-isopropylamino-s-triazine, 2-chloro-4,6-bis(isopropylamino)-s-triazine, 2-methoxy-4,6-bis(ethylamino)-s-triazine, 2-methoxy-4-ethylamino-6-isopropylamino-s-triazine, and 2-methylmercapto-4-(2-methoxyethyl- amino)-6-isopropylamino-s-triazine.

## Diphenyl Ether Derivatives

2,4-dichloro-4'-nitrodiphenyl ether, 2,4,6-trichloro-4'-nitrodiphenyl ether, 2,4-dichloro-6-fluoro-4' -nitrodiphenyl ether, 2-chloro-4-trifluoromethyl-3'-carboxy-4'-nitrodiphenyl-ether and its salt and ester derivatives, 3-methyl-4'-nitrodiphenyl ether, 3,5-dimethyl-4'-nitrodiphenyl ether, 2,4'-dinitro-4-trifluoromethyldiphenyl ether, 2,4-dichloro-3'-methoxy-4'-nitrodiphenyl ether, and sodium 5-(2-chloro-4-trifluoro- methylphenoxy)-2-nitrobenzoate.

Anilides

N-(3,4-dichlorophenyl) propionamide, N-(3,4-dichlorophenyl) methacrylamide, N-(3-chloro-4-methylphenyl)-2-methylpentamide, N-(3,4-dichlorophenyl) trimethylacetamide, N-(3,4-dichlorophenyl) B.B-dimethylvaleramide, N-isopropyl-N-phenylchloroacetamide and N-n-methoxy-methyl-N-(2,6-diethylphenyl)chloroacetamide, and 2-chloro-N-(2-ethyl-6-methylphenyl)-N-(2-methoxy-1-methylethyl) acetamide.

Uracils

5-bromo-3-s-butyl-6-methyluracil, 5-bromo-3-cyclohexyl-1,6-dimethyluracil,3-cyclohexyl-5,6-trimethyleneuracil, 5-bromo-3-isopropyl-6-methyluracil and 3-tert-butyl-5-chloro-6-methyluracil.

Nitriles

2,6-dichlorobenzonitrile, diphenylacetonitrile, 3,5-dibromo-4-hydroxybenzonitrile, and 3,5-diiodo-4-hydroxybenzonitrile.

Other Organic Herbicides

2-chloro-N,N-diallylacetamide, N-(1,1-dimethyl-2-propynyl)-3,5-dichlorobenzamide, maleic hydrazide, 3-amino-1,2,4-triazole, monosodium methanearsonate, N,N-diallyl-2-chloroacetamide, disodium methanearsonate, N,N-dimethyl-B.B-diphenylacetamide, N,N-di(n-propyl)-2,6-dinitro-4-trifluoromethylaniline, N,N-di(n-propyl)-2,6-dinitro-4-methylaniline, N,N-di(n-propyl)-2,6-dinitro-4-methylsulfonylaniline, $N^3$,$N^3$-di-n-propyl-2,4-dinitro-6-trifluoromethyl-m-phenylenediamine, 3,5-dinitro-$N^4$,$N^4$-dipropylsulfanilamide, 4-isopropyl-2,6-dinitro-N,N-dipropylaniline, N-(2-chloroethyl)-2,6-dinitro-N-propyl-4-trifluoromethylaniline, O-(2,4-dichlorophenyl)-O-methylisopropylphosphoramidothioate, 4-amino-3,5,6-trichloropicolinic acid, 2,3-dichloro-1,4-naphthoquinone, di-(methoxythiocarbonyl)disulfide, and 3-isopropyl-1H-2,1,3-benzothiadiazine(4)3H-one-2,2 dioxide.

The following examples illustrate the products of this invention and methods for preparing them.

Example 1

A solution of 9.1g (0.06 mole) of 3,4,5,6-tetrahydrophthalic anhydride and 11.1g (0.06 mole) of methyl 2-chloro-5-aminobenzoate was dissolved in glacial acetic acid (30 ml) and vigorously refluxed for one hour. The reaction mixture was cooled to room temperature and the crude product collected by filtration, washed with water and then dried to afford 14.9g of N-[3-methoxycarbonyl-4-chlorophenyl]-3,4,5,6-tetrahydrophthalimide - m.p. 113°-115°C.

By following substantially the procedure described in Example 1 other compounds, wherein Z contains a carbonyl group attached directly to the benzene ring, may be prepared in a similar manner. The following table together with the structure identified as "Formula III" illustrate compounds prepared by this method.

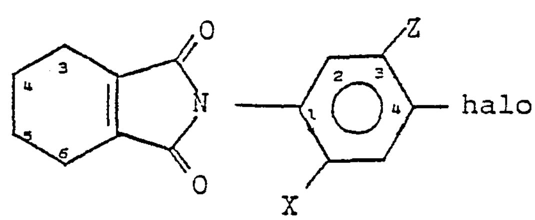

Formula (III)

| Example No. | X | Halo | Z | m.p. °C | Molecular Formula |
|---|---|---|---|---|---|
| 1 | H | Cl | $-CO_2CH_3$ | 113-115 | $C_{16}H_{14}ClNO_4$ |
| 2 | H | Cl | $-CO_2H$ | 239-241 | $C_{15}H_{12}ClNO_4$ |
| 3 | H | Cl | $-CO_2CH_2CO_2Me$ | 113-117 | $C_{18}H_{16}ClNO_6$ |
| 4 | H | Cl | $-CO_2CH(Me)CO_2Me$ | [oil] | $C_{19}H_{18}ClNO_6$ |
| 5 | H | Cl | $-CO_2CH(Et)CO_2Et$ | [oil] | $C_{21}H_{22}ClNO_6$ |
| 6 | H | Cl | $-CO_2(CH_2)_3CO_2Et$ | [oil] | $C_{21}H_{22}ClNO_6$ |

Example 7 - [N- 3-(3-Ethoxycarbonyl-n-propoxy)-4-chlorophenyl] 3,4,5,6-tetrahydrophthalimide

Step A: N-(3-Hydroxy-4-chlorophenyl)-3,4,5,6-tetrahydrophthalimide

Employed: 47.5 g of 3-hydroxy-4-chloroaniline (.33 mol); 38.2 g of 3,4,5,6-tetrahydrophthalic anhydride (.25 mol) and 350 ml of glacial acetic acid.

Procedure:

To a 500 ml round bottom flask the crude aniline was added together with the anhydride and glacial acetic acid. The mixture was refluxed for 2 days; after which water was added and the reaction mixture was then poured into a separatory funnel, concentrated HCl was added and the mixture was extracted with ether and dried over anhydrous $MgSo_4$. Total crude yield was 42.4 g. recrystallization, a 1.0 g sample from methanol and water provided 0.7 g of product, m.p., 220°-225°C. NMR and I.R. analyses agree with the expected product structure.

Step B: N-[3-(3-Ethoxycarbonylpropoxy)-4-chlorophenyl]-3,4,5,6-tetrahydrophthalimide

To a 100 ml round bottom flask equipped with a reflux condenser, magnetic stirrer and heating mantle, 5.0 g of potassium carbonate, 5.0 g of N-(3-hydroxy-4-chlorophenyl)-3,4,5,6-tetrahydrophthalimide and 80 ml of methyl ethyl ketone were added. 2.9 ml of ethyl 4-bromobutyrate was then added and the mixture refluxed. Thin layer chromatography showed that the reaction was incomplete. More ethyl 4-bromobutyrate (2-3 ml) was added together with potassium carbonate (2.0 g) and the mixture was refluxed for an additional 1 hour. Solvent was removed under reduced pressure and ether was added to the residue. The residue was washed successively with 30 ml of 2 M sodium hydroxide, 15 ml of 6 M sodium hydroxide and 20 ml of saturated salt solution. Ether was removed to obtain 6 g of N-[3-(3-ethoxycarbonylpropoxy)-4-chloro-phenyl]-3,4,5,6-tetrahydrophthalimide. NMR and IR confirm the identity of the product obtained.

In substantially the same manner as described in example 7 and by employing the appropriate starting materials, the following products were prepared.

| Example No. | X | Halo | Z | m.p.°C |
|---|---|---|---|---|
| 8 | H | Cl | $-OCOCH_3$ | oil |
| 9 | H | Cl | $-OCH_2CN$ | 110-118 |
| 10 | H | Cl | $-OCH_2SCH_3$ | oil |
| 11 | H | Cl | $-OCH_2COCH_3$ | 100-105 |
| 12 | H | Cl | $-OCH_2\overbrace{CH-CH_2}^{O}$ | -- |
| 13 | H | Cl | $-OCH_2CO_2Me$ | oil |
| 14 | Cl | Cl | $-OCH(Me)CO_2Me$ | oil |
| 15 | H | Cl | $-OCH_2CH=CHCO_2Me$ | oil |
| 16 | H | Cl | $-O(CH_2)_3CO_2Et$ | oil |
| 17 | H | F | $-OCH(Me)CN$ | -- |
| 18 | H | Cl | $-OCH_2CHO$ | -- |
| 19 | H | Cl | $-OCH_2OCH_3$ | -- |
| 20 | H | Br | $-OCH_2CH_2OCH_3$ | -- |
| 21 | Br | Cl | $-OCH_2CH_2Cl$ | -- |
| 22 | H | Cl | $-CO_2Na$ | -- |
| 23 | F | Cl | $-OCH_3CN$ | -- |
| 24 | H | Cl | $-OCH(CH_3)CN$ | oil |
| 25 | H | Cl | $-OCH_2CH_2OCH_3$ | oil |
| 26 | H | Cl | $-OCH_2CH_2Cl$ | 94-100 |
| 27 | H | Cl | $-OCH(CH_3)COCH_3$ | 86-92 |
| 28 | H | Cl | $-OCH(CH_3)SCH_3$ | oil |
| 29 | H | Cl | $-OCH(CH_2CH_3)CN$ | oil |
| 30 | H | Br | $-OCH(CH_3)CN$ | 113-116 |
| 31 | H | Cl | $-OCH_2CF_3$ | 91.5-96 |
| 32 | H | $CH_3$ | $-OCH(CH_3)CN$ | 149 |

Example 33 - N-(3-Ethylamino-4-chlorophenyl)-3,4,5,6-tetrahydrophthalimide

Used:

2.5 g of 3-ethylamino-4-chloroaniline;

2.1 g of 3,4,5,6-tetrahydrophthalic anhydride and

2-3 ml of glacial acetic acid.

Procedure

In a 50 ml round bottomed flask, the above ingredients were mixed and refluxed overnight under nitrogen.

Water was added and the mixture was extracted with 3 portions of methylene chloride ($CH_2Cl_2$). The mixture was then washed three times with 2 M NaOH (to remove acetic acid), then washed with brine. The $CH_2Cl_2$ was evaporated to afford 4.0 g of crude product which was recrystallized from a solvent consisting of ethyl acetate and hexane to yield 2.8 g of product as a green solid, m.p. 120-127°C.

Example 34 - N-(3-Dimethylamino-4-chlorophenyl)-3,4,5,6-tetrahydrophthalimide

Step A: N-(3-nitro-4-chlorophenyl)-3,4,5,6-tetrahydrophthalimide

To a stirred mixture of 25 g of 3,4,5,6-tetrahydrophthalic anhydride and 150 ml of glacial acetic acid, under $N_2$, 31.2 g of 4-chloro-3-nitroaniline was added and the mixture heated to reflux overnight. The reaction mixture was cooled to room temperature and the resulting precipitate filtered and washed with hexane to yield 45 g of product.

Step B: N-(3-Amino-4-chlorophenyl)-3,4,5,6-tetrahydrophthalimide

To a solution of 40 g of N-(3-nitro-4-chlorophenyl)-3,4,5,6-tetrahydrophthalimide in 900 ml. of methanol and 100 ml of ethyl acetate in a hydrogenation bottle 0.5 g $PtO_2$ was added. The bottle was purged with hydrogen then pressurized to 35153 kg/$m^2$ (50 psi) and shaken until the required amount of $H_2$ was used. The reaction mixture was filtered through celite, washed well with $CH_2Cl_2$ and the combined organic phases concentrated in vacuo to yield 29 g of the product. This product was used without further purification in subsequent reactions.

Step C: N-(3-Dimethylamino-4-chlorophenyl)-3,4,5,6-tetrahydrophthalimide

To a stirred solution of 3.5 g of N-(3-amino-4-chlorophenyl)-3,4,5,6-tetrahydrophthalimide and 3.5 g powdered $K_2CO_3$ in 70 ml acetone under $N_2$, 5 ml $CH_3I$ was added and the mixture heated to reflux overnight. Two additional portions of $CH_3I$ were added (5 ml each) during the course of the reaction. The reaction mixture was cooled to room temperature, filtered and the solvent removed under vacuum. The crude product was dissolved in $CH_2Cl_2$ and passed through a plug of alumina and the solvent was then evaporated. The partially purified product was further purified by HPLC eluting with a mixture of 33% by volume of ethyl acetate in hexane, to give the product - m.p. 124-7°C.

The following procedure can be employed to prepare other amino and substituted amino compounds:

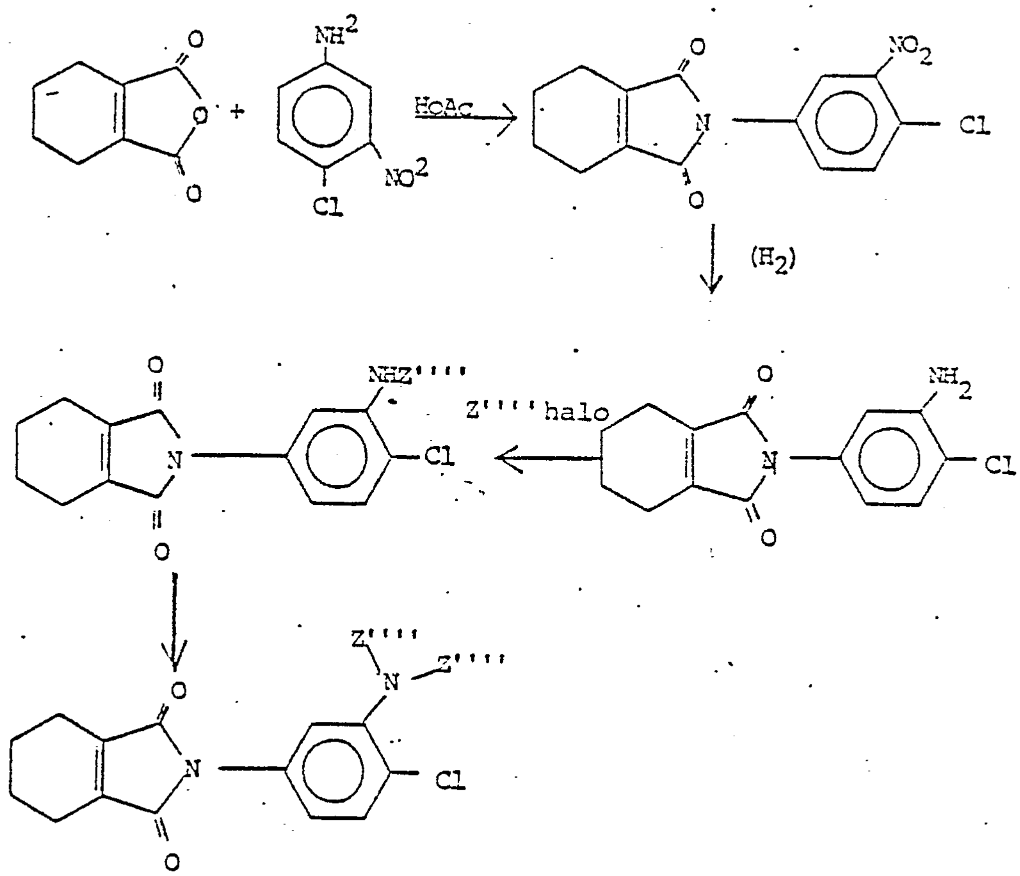
HOAc
(H2)
Z''''halo

| Example No. | Z'''' | Z'''' | m.p.°C |
|---|---|---|---|
| 35 | H | $NH_2$ | -- |
| 36 | $CH_3$ | H | -- |
| 37 | $CH_3CO-$ | H | -- |
| 38 | $CH_3O-$ | H | -- |
| 39 | $CH_3OCO-$ | H | -- |
| 40 | $CH_3NHCO-$ | H | -- |
| 41 | $CH_3CH_2CO-$ | H | -- |
| 42 | $CH_3SO_2-$ | H | -- |
| 43 | $CH_3OCH_2N(CH_3)CO-$ | H | -- |
| 44 | $CH{=}CHCH_2-$ | $CH{=}CHCH_2-$ | oil |
| 45 | $C_2H_5-$ | $CH{=}CHCH_2-$ | -- |
| 46 | $C_3H_5-$ | $C{\equiv}CCH_2-$ | -- |
| 47 | $C_2H_5-$ | H | 130-137 |
| 48 | $ClCH_2CO-$ | H | 111-118 |

Example 49 - N-[3-(2-Cyanoethyl)-4-chlorophenyl]-3,4,5,6-tetrahydrophthalimide

Step A: 2-Chloro-5-nitrocinnamylnitrile

To a slurry of 20.5 g of 2-chloro-5-nitroaniline in 22 ml $H_2O$ and 45 ml of conc. HCl at room temperature was slowly added a conc. aqueous solution of $NaNO_2$ (9.0g $NaNO_2$). The slurry was stirred at room temperature for 30 minutes. The resulting diazonium salt was added slowly and portionwise to a stirred room temperature mixture of 10 ml of acetic acid/80 ml acetone/30 ml 25% aqueous NaOH containing 3.5 g CuCl and 9.4 ml acrylonitrile. After addition of the diazonium salt was complete, the slurry was warmed to $35^{o}$-$45^{o}C$ for 1.5 hours during which time $N_2$ evolution ceased. The reaction mixture was washed well with $CH_2Cl_2$, the $CH_2Cl_2$ washings combined, filtered, and dried over $MgSO_4$. The solvent was evaporated after filtration to yield 14g of the intermediate product. All of this material was added to refluxing $Et_3N$. The reaction mixture was heated to reflux for an additional 15 min, cooled to room temperature and $CH_2Cl_2$ added. The organic phase was washed with cold dilute $H_2SO_4$ until all of the $Et_3N$ had been removed from the organic phase. The $CH_2Cl_2$ solution was dried over $MgSO_4$, filtered and the solvent removed to yield 10.2g of the desired product. This material was used in subsequent reactions without further purification.

Step B: 3-(2-Cyanoethyl)-4-chloroaniline

To a solution of 10.2g of 2-chloro-5-nitro cinnamylnitrile in 150 ml 1:1 ethyl acetate/methanol was added 0.5g $PtO_2$ in a hydrogenation bottle. The bottle was purged with $H_2$, pressurized to 35153 $kg/m^2$ (50 psi) and shaken until the calculated amount of $H_2$

had been used. The reaction mixture was filtered through "Celite" and washed well with ethyl acetate. Concentration of the solution gave 7.9 g of the desired aniline. This product was used in the subsequent reaction without further purification.

Step C: N-[3-(2-Cyanoethyl)-4-chlorophenyl]-3,4,5,6-tetrahydrophthalimide

To a stirred slurry of 5.0g of 3,4,5,6-tetrahydrophthalic anhydride in 5 ml of glacial acetic acid under $N_2$ 7.5g of 3-(2-cyanoethyl)-4-chloroaniline was added and the mixture heated to reflux for 2 hours. The reaction mixture was cooled to room temperature, water was added together with several ml of conc. HCl and the mixture extracted with ether. The combined ether extracts were washed with saturated brine and then dried over $MgSO_4$. Filtration and removal of the solvent yielded 8.9g of a brown oil. The oil was dissolved in $CH_2Cl_2$, passed through a plug of alumina, the solvent removed, and then further purified by HPLC eluting with 25% by volume of ethyl acetate in hexane to yield the product as a yellow solid, m.p. 87-97$^{o}$C.

Test Procedure

This example shows the procedure for determining herbicidal activity of the compounds of the invention by employing the following representative weed species:

| | | | Approx. No. Seeds |
|---|---|---|---|
| Monocots: | Barnyardgrass | (Echinochloa crusgalli) | 25 |
| | Downybrome | (Bromus tectorum) | 20 |
| | Foxtail | (Setaria spp) | 25 |
| | Johnsongrass | (Sorghum Halepenese) | 25 |
| | Nutsedge | (Cyperus esculentus) | 5 |
| | Wild Oat | (Avena fatua) | 20 |
| Dicots: | Cocklebur | (Xanthium pensylvanicum) | 3 |
| | Marigold | (Tagetes spp) | 15 |
| | Morning Glory | (Ipomoea spp) | 10 |
| | Tomato | (Lycopersicon esculentum) | 15 |
| | Velvetleaf | (Abutilon theophrasti) | 15 |
| | Sicklepod | (Cassia obtusifolia) | 6 |

The following test procedure is employed. Seeds of the above species are planted in soil in trays 17.78 x 26.67 x 7.62 cm (approx. 7" x 10 1/2" x 3"). For preemergence tests, the trays were sprayed with the rest compound immediately after planting. For postemergence tests, the seeds were allowed to germinate and after growing in the greenhouse for

two weeks, the growing plants were treated with the test compound. The compound to be evaluated was dissolved in acetone or water and sprayed over the trays using a carrier volume equivalent to 468 l/ha (50 gallons per acre) at the rate of application in kg/ha (pounds per acre, lb/A) specified in the table. About two weeks after application of the test compound, the state of growth of the plants is observed and the phytotoxic effect of each compound determined as follows: each species is evaluated on a scale of 1-100 in which 0 = no activity and 100 = total kill and the results for the monocots (M) and dicots (D) separately averaged. The results obtained for the compounds of the invention tested at 2.24, 0.56 and 0.14 kg/ha (2 lb/A, 1/2 lb/A and 1/8 lb/A) indicate that the compounds are selective herbicides.

HERBICIDAL DATA

| Exam. No. | Preemergence 1/2 lb./A. MONOCOT | DICOT | Preemergence 2 lbs./A. MONOCOT | DICOT | Postemergence 1/2 lb./A. MONOCOT | DICOT | Postemergence 2 lbs./A. MONOCOT | DICOT |
|---|---|---|---|---|---|---|---|---|
| 1 | 0 | 23 | 28 | 40 | 5 | 65 | 10 | 78 |
| 2 | 2 | 4 | 2 | 20 | 8 | 28 | 3 | 70 |
| 3 | 0 | 0 | 0 | 0 | 0 | 58 | 3 | 86 |
| 4 | 5 | 6 | 10 | 66 | 27 | 98 | 36 | 100 |
| 5 | 0 | 0 | 6 | 0 | 0 | 32 | 17 | 96 |
| 6 | 0 | 0 | 0 | 0 | 0 | 84 | 0 | 86 |
| 8 | 8 | 32 | 17 | 61 | 16 | 33 | 19 | 47 |
| 9 | 21 | 72 | 52 | 83 | 11 | 92 | 21 | 99 |
| 10 | 31 | 78 | 54 | 79 | 9 | 79 | 19 | 93 |
| 11 | 39 | 78 | 46 | 83 | 5 | 52 | 17 | 86 |
| 12 | 11 | 43 | 26 | 60 | 4 | 53 | 5 | 81 |
| 14 | 2 | 34 | 18 | 46 | 2 | 56 | 4 | 56 |
| 16 | 3 | 63 | 5 | 78 | 5 | 76 | 4 | 96 |
| 19 | 57 | 84 | 89 | 100 | 26 | 100 | 58 | 100 |
| 25 | 46 | 50 | 69 | 68 | 23 | 67 | 30 | 83 |
| 26 | 40 | 42 | 71 | 87 | 18 | 78 | 34 | 85 |
| 27 | 32 | 63 | 70 | 83 | 33 | 78 | 43 | 78 |
| 28 | 42 | 95 | 73 | 98 | 22 | 89 | 28 | 96 |
| 29 | 49 | 75 | - | - | 8 | 65 | - | - |
| 30 | 53 | 83 | 64 | 98 | 6 | 65 | 8 | 76 |
| 31 | 12 | 24 | 38 | 64 | 8 | 36 | 13 | 38 |
| 32 | 42 | 62 | 48 | 85 | 3 | 40 | 9 | 63 |
| 47 | 43 | 75 | 59 | 73 | 27 | 76 | 29 | 88 |
| 48 | 0 | 3 | 0 | 3 | 2 | 2 | 3 | 3 |
| 49 | 7 | 37 | 31 | 60 | 2 | 50 | 11 | 51 |

Compounds disclosed in U.S. Serial No. 278,241 upon which this application is in part based are, *inter alia*, those of the formula:

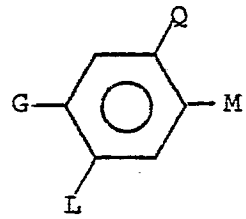

(IV)

wherein G is a 5-membered heterocyclic radical; L is hydrogen or halo; M is halo, cyano, $(C_1-C_4)$alkoxy, $(C_1-C_4)$alkylthio or nitro, and Q is carboxy, alkoxy alkoxy alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy, alkoxycarbonylalkenyloxy, a 5- to 6-membered heterocyclic, formyl alkoxy, haloalkoxy, mononuclear arylcarbonylalkoxy, oxiranylalkoxy, amino, monoalkyl amino, dialkyl amino, hydrazino, monoalkyl hydrazino, dialkyl hydrazino, acylamino, alkoxyamino, alkoxy carbonyl amino, monoalkyl amino carbonyl amino, dialkylaminocarbonyl amino, N-alkyl-N-alkoxyalkylaminocarbonylamino, haloalkylcarbonylamino, N-alkyl sulfonylcarbonyl, N-alkyl-sulfonylamino, monoalkenylamino, dialkenylamino, mono-alkynyl amino, dialkynylamino, N-alkyl-N-alkenylamino, or N-alkyl-N-alkynyl amino and agronomically acceptable salts, esters and amides thereof.

The invention also includes within its scope compounds of the above formula (IV) wherein G is a 5-membered heterocyclic radical, L is hydrogen or halo, M is halo and Q is carboxy, alkoxycarbonyl having up to 4 carbon atoms in the alkoxy moiety, alkoxycarbonylalkoxycarbonyl having in each alkoxy moiety which may be the same

or different -1,2,3 or 4 carbon atoms, alkoxyalkoxycarbonyl having in each alkoxy moiety which may be same or different -1,2,3 or 4 carbon atoms, acyl$(C_1-C_4)$-alkoxy, cyano$(C_1-C_4)$alkoxy, $(C_1-C_4)$alkylthio$(C_1-C_4)$-alkoxy, alkoxycarbonylalkoxy having in each alkoxy moiety which may be the same or different -1,2,3 or 4 carbon atoms, alkoxycarbonyl$(C_3-C_5)$alkenyloxy having up to 4 carbon atoms in the alkoxy moiety, formyl$(C_1-C_4)$alkoxy, halo$(C_1-C_4)$alkoxy, mononuclear arylcarbonyl$(C_1-C_4)$-alkoxy, oxiranyl$(C_1-C_4)$alkoxy, mono- or di-$(C_1-C_4)$alkylamino, mono- or di-$(C_1-C_4)$alkylhydrazino, $(C_1-C_4)$alkoxyamino, alkoxycarbonylamino having up to 4 carbon atoms in the alkoxy moiety, N-$(C_1-C_4)$alkyl-N-$(C_1-C_4)$alkoxy-$(C_1-C_4)$alkylaminocarbonylamino, haloalkylcarbonylamino having up to 4 carbon atoms in the alkyl moiety, N-$(C_1-C_4)$alkylsulfonylamino, mono- or di-$(C_3-C_5)$alkenylamino, mono- or di-$(C_3-C_5)$alkynylamino, N-$(C_1-C_4)$alkyl-N-$(C_3-C_5)$alkenylamino or N-$(C_1-C_4)$alkyl-N-$(C_3-C_5)$-alkynylamino and agronomically acceptable salts, esters and amides thereof.

In conclusion there is presented a preparative example of a compound having a structure somewhat similar to that given in formula I.

Example 50 - N-(4-chlorophenyl)-3-oxa-6-thia-3,4,5,6-tetrahydrophthalimide

To a stirred solution of 1.6 g sodium methoxide in 50 ml methanol, 1.17g of 2-mercaptoethanol was added at room temperature. The solution was stirred for 15 min. then 4.5 g of N-(4-chlorophenyl)-dichloromaleimide was added in one portion. The reaction was completed in one hour. The solvent was removed and the resulting solid triturated with ether. The combined ether washings were extracted with 2 x 40 ml portions of water and dried over $Na_2SO_4$. After filtration through diatomaceous earth ("Celite") and removal of the solvent, a yellow oil was obtained which was then purified by HPLC eluting with 30% by volume of ethyl acetate in hexane. This produced 0.5 g of the product as a pale yellow solid; m.p. 158°C.

CLAIMS:

1. A compound of the formula

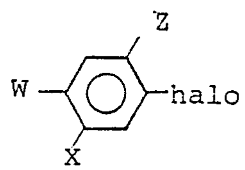 where W is a substituted

heterocyclic selected from:

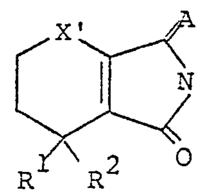

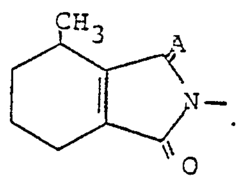

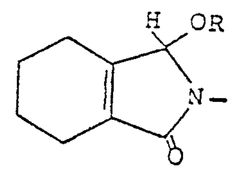

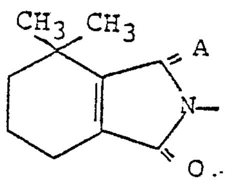

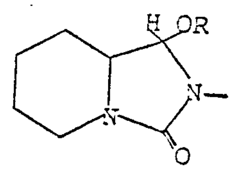

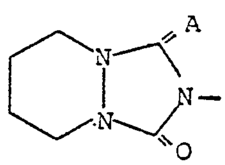

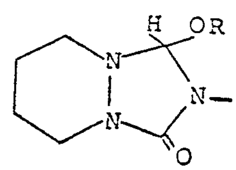

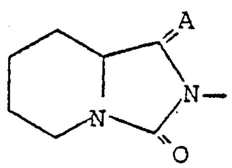

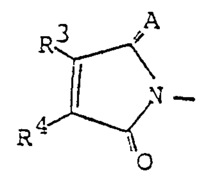

wherein A is O or S; halo is chloro, bromo or fluoro; $R^1$ and $R^2$ are the same or different radicals selected from hydrogen or $(C_1-C_4)$alkyl; X is hydrogen or halo; $X^1$ is methylene, O or S; Z is carboxy, alkoxyalkoxy, alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy, alkoxycarbonylalkenyloxy, a 5- to 6-membered heterocyclic, formylalkoxy, haloalkoxy, mononuclear arylcarbonylalkoxy, oxiranylalkoxy, amino, monoalkyl amino, dialkylamino, hydrazino, monoalkylhydrazino, dialkylhydrazino, acylamino, alkoxyamino, alkoxycarbonylamino, mono- or dialkylcarbonylamino, N-alkyl-N-alkoxyalkylaminocarbonylamino, haloalkylcarbonylamino, N-alkylsulfonylaminocarbonyl, N-alkylsulfonylamino, monoalkenylamino, dialkenylamino, monoalkynylamino, dialkynylamino, N-alkyl-N-alkenylamino, or N-alkyl-N-alkynylamino; R is hydrogen or $(C_1-C_4)$alkyl and $R^3$ and $R^4$ are the same or different and are selected from hydrogen, $(C_1-C_4)$alkyl, $(C_3-C_5)$alkenyl or $(C_3-C_5)$-alkynyl.

2. A compound according to claim 1 of the formula:

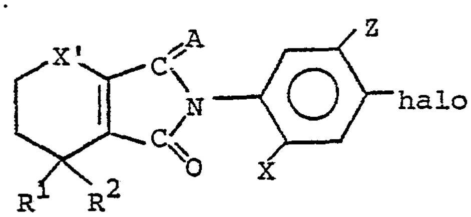

wherein A is O or S; $R^1$ and $R^2$ are the same or different radicals selected from hydrogen or $(C_1-C_4)$alkyl; X is hydrogen or halo; $X^1$ is methylene, O or S; and Z is carboxy, alkoxyalkoxy, alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy,

alkoxycarbonylalkenyloxy, a 5- to 6-membered heterocyclic, formylalkoxy, haloalkoxy, mononuclear arylcarbonylalkoxy, oxiranylalkoxy, amino, monoalkyl amino, dialkylamino, hydrazino, monoalkylhydrazino, dialkylhydrazino, acylamino, alkoxyamino, alkoxycarbonylamino, monoalkylaminocarbonylamino, dialkylaminocarbonylamino, N-alkyl-N-alkoxyaminocarbonylamino, haloalkylcarbonylamino, N-alkylsulfonylaminocarbonyl, N-alkyl-sulfonylamino, monoalkenylamino, dialkenylamino, monoalkynylamino, dialkynylamino, N-alkyl-N-alkenyl amino, or N-alkyl-N-alkynylamino.

3. A compound according to claim 2 of the formula:

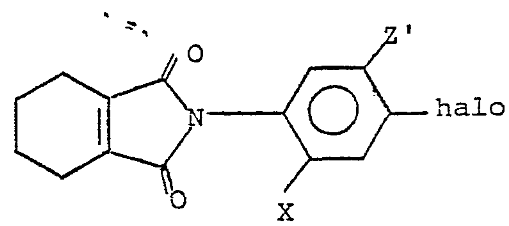

wherein halo is chloro or bromo; X is halo or hydrogen; and Z' is carboxy, alkoxycarbonyl having up to 4 carbon atoms in the alkoxy moiety, $(C_1-C_4)$alkoxyalkoxycarbonyl having up to 4 carbon atoms in the alkoxy moiety, cyano-$(C_1-C_4)$alkoxy, $(C_1-C_4)$alkylthio$(C_1-C_4)$alkoxy, acetyl-$(C_1-C_4)$alkoxy, $(C_1-C_4)$alkoxy$(C_1-C_4)$alkoxy, halo$(C_1-C_4)$-alkoxy, formyl$(C_1-C_4)$alkoxy, or mono$(C_1-C_4)$alkylamino.

4. A compound according to claim 4, wherein Z' is cyano$(C_1-C_4)$alkoxy, $(C_1-C_4)$alkylthio$(C_1-C_4)$alkoxy or acetyl$(C_1-C_4)$alkoxy, and X is hydrogen.

5. A compound selected from N-[3-(1-cyanoethoxy)-4-chlorophenyl]3,4,5,6-tetrahydrophthalimide, N-[3-(1-methylthioethoxy)-4-chlorophenyl]3,4,5,6-tetrahydrophthalimide, N-[3-(1-cyanoethoxy)-4-bromophenyl]3,4,5,6-tetrahydrophthalimide, N-[3-(1-acetylethoxy)-4-chlorophenyl]3,4,5,6-tetrahydrophthalimide, N-[3-(1-cyanopropoxy)-4-chlorophenyl]3,4,5,6-tetrahydrophthalimide, N-[3-(1-cyanoethoxy)-4-fluorophenyl]3,4,5,6-tetrahydrophthalimide.

6. A compound according to claim 1 of the formula

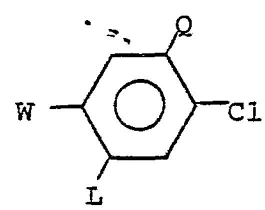

wherein W is as defined in claim 1, L is hydrogen or halo and Q is carboxy, alkoxy alkoxy alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy, alkoxycarbonylalkenyloxy, a 5- to 6-membered heterocyclic, formyl alkoxy, haloalkoxy, mononuclear arylcarbonylalkoxy, oxiranylalkoxy, amino, monoalkyl amino, dialkyl amino, hydrazino, monoalkyl hydrazino, dialkyl hydrazino, acylamino, alkoxyamino, alkoxy carbonyl amino, monoalkyl amino carbonyl amino, dialkylaminocarbonyl amino, N-alkyl-N-alkoxyaminocarbonylamino, haloalkylcarbonylamino, N-alkyl sulfonylcarbonyl, N-alkylsulfonylamino, monoalkenylamino, dialkenylamino, monoalkynyl amino, dialkynylamino, N-alkyl-N-alkenylamino,

or N-alkyl-N-alkynyl amino and agronomically acceptable salts, esters and amides thereof.

7. A method for controlling weeds which comprises applying to the weed or a growth medium therefor a herbicidally effective amount of a composition according to any of claims 1 to 5.

8. A method for controlling weeds which comprises applying to the weed or a growth medium therefor a herbicidally effective amount of a composition according to claim 6.

Austrian Claim
81-16A
0068822



CLAIMS:

1. A herbicidal composition containing an active herbicidal component and an agronomically acceptable carrier or diluent therefor, wherein the active component comprises one or more compounds of the formula

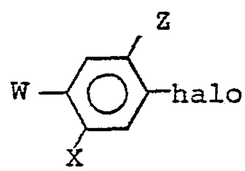

wherein W is a substituted heterocyclic selected from:

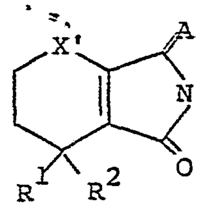

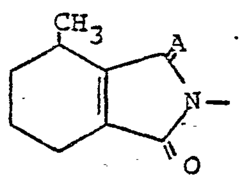

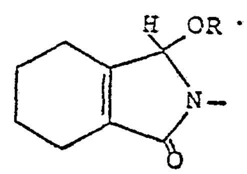

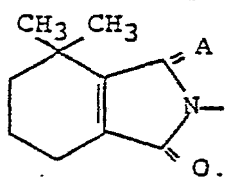

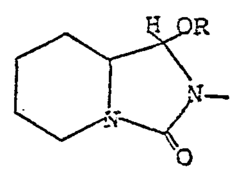

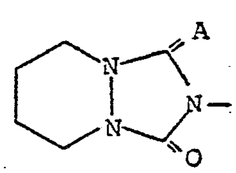

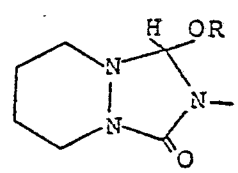

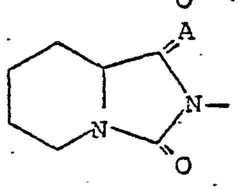

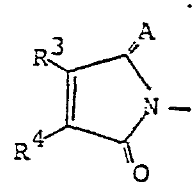

wherein A is O or S; halo is chloro, bromo or fluoro; $R^1$ and $R^2$ are the same or different radicals selected from hydrogen or $(C_1-C_4)$alkyl; X is hydrogen or halo; $X^1$ is methylene, O or S; Z is carboxy, alkoxyalkoxy, alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy, alkoxycarbonylalkenyloxy, a 5- to 6-membered heterocyclic, formylalkoxy, haloalkoxy, mononuclear arylcarbonylalkoxy, oxiranylalkoxy, amino, monoalkyl amino, dialkylamino, hydrazino, monoalkylhydrazino, dialkylhydrazino, acylamino, alkoxyamino, alkoxycarbonylamino, mono- or dialkylcarbonylamino, N-alkyl-N-alkoxyalkylaminocarbonylamino, haloalkylcarbonylamino, N-alkylsulfonylaminocarbonyl, N-alkylsulfonylamino, monoalkenylamino, dialkenylamino, monoalkynylamino, dialkynylamino, N-alkyl-N-alkenylamino, or N-alkyl-N-alkynylamino; R is hydrogen or $(C_1-C_4)$alkyl and $R^3$ and $R^4$ are the same or different and are selected from hydrogen, $(C_1-C_4)$alkyl, $(C_3-C_5)$alkenyl or $(C_3-C_5)$-alkynyl.

2. A composition according to claim 1 wherein the active component comprises one or more compounds of the formula:

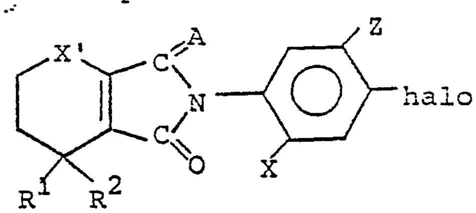

wherein A is O or S; $R^1$ and $R^2$ are the same or different radicals selected from hydrogen or $(C_1-C_4)$alkyl; X is hydrogen or halo; $X^1$ is methylene, O or S; and Z is carboxy, alkoxyalkoxy, alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy,

alkoxycarbonylalkenyloxy, a 5- to 6-membered heterocyclic, formylalkoxy, haloalkoxy, mononuclear arylcarbonylalkoxy, oxiranylalkoxy, amino, monoalkyl amino, dialkylamino, hydrazino, monoalkylhydrazino, dialkylhydrazino, acylamino, alkoxyamino, alkoxycarbonylamino, monoalkylaminocarbonylamino, dialkylaminocarbonylamino, N-alkyl-N-alkoxyaminocarbonylamino, haloalkylcarbonylamino, N-alkylsulfonylaminocarbonyl, N-alkyl-sulfonylamino, monoalkenylamino, dialkenylamino, monoalkynylamino, dialkynylamino, N-alkyl-N-alkenyl amino, or N-alkyl-N-alkynylamino.

3. A composition according to claim 2 wherein the active component comprises one or more compounds of the formula:

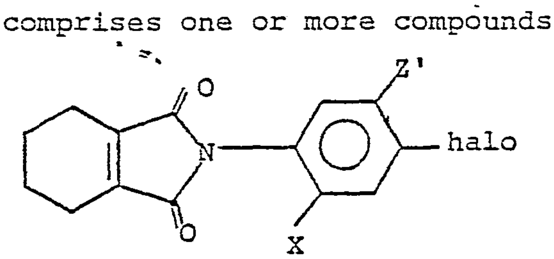

wherein halo is chloro or bromo; X is halo or hydrogen; and Z' is carboxy, alkoxycarbonyl having up to 4 carbon atoms in the alkoxy moiety, $(C_1-C_4)$alkoxyalkoxycarbonyl having up to 4 carbon atoms in the alkoxy moiety, cyano-$(C_1-C_4)$alkoxy, $(C_1-C_4)$alkylthio$(C_1-C_4)$alkoxy, acetyl-$(C_1-C_4)$alkoxy, $(C_1-C_4)$alkoxy$(C_1-C_4)$alkoxy, halo$(C_1-C_4)$-alkoxy, formyl$(C_1-C_4)$alkoxy, or mono$(C_1-C_4)$alkylamino.

4. A composition according to claim 4, wherein Z' is cyano$(C_1-C_4)$alkoxy, $(C_1-C_4)$alkylthio$(C_1-C_4)$alkoxy or acetyl$(C_1-C_4)$alkoxy, and X is hydrogen.

5. A composition according to claim 4 containing an active component selected from N-[3-(1-cyanoethoxy)-4-chlorophenyl]3,4,5,6-tetrahydrophthalimide, N-[3-(1-methylthioethoxy)-4-chlorophenyl]3,4,5,6-tetrahydrophthalimide, N-[3-(1-cyanoethoxy)-4-bromophenyl]3,4,5,6-tetrahydrophthalimide, N-[3-(1-acetylethoxy)-4-chlorophenyl]3,4,5,6-tetrahydrophthalimide, N-[3-(1-cyanopropoxy)-4-chlorophenyl]3,4,5,6-tetrahydrophthalimide, N-[3-(1-cyanoethoxy)-4-fluorophenyl]3,4,5,6-tetrahydrophthalimide.

6. A composition according to claim 1 wherein the active component comprises one or more compounds of the formula

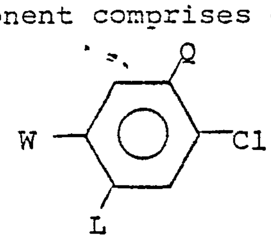

wherein W is as defined in claim 1, L is hydrogen or halo and Q is carboxy, alkoxy alkoxy alkoxycarbonyl, alkoxycarbonylalkoxycarbonyl, alkoxyalkoxycarbonyl, acyloxy, acylalkoxy, cyanoalkoxy, alkylthioalkoxy, alkoxycarbonylalkoxy, alkoxycarbonylalkenyloxy, a 5- to 6-membered heterocyclic, formyl alkoxy, haloalkoxy, mononuclear arylcarbonylalkoxy, oxiranylalkoxy, amino, monoalkyl amino, dialkyl amino, hydrazino, monoalkyl hydrazino, dialkyl hydrazino, acylamino, alkoxyamino, alkoxy carbonyl amino, monoalkyl amino carbonyl amino, dialkylaminocarbonyl amino, N-alkyl-N-alkoxyaminocarbonylamino, haloalkylcarbonylamino, N-alkyl sulfonylcarbonyl, N-alkylsulfonylamino, monoalkenylamino, dialkenylamino, monoalkynyl amino, dialkynylamino, N-alkyl-N-alkenylamino,

or N-alkyl-N-alkynyl amino and agronomically acceptable salts, esters and amides thereof.

7. A method for controlling weeds which comprises applying to the weed or a growth medium therefor a herbicidally effective amount of a composition according to any of claims 1 to 5.

8. A method for controlling weeds which comprises applying to the weed or a growth medium therefor a herbicidally effective amount of a composition according to claim 6.